# EUROPEAN PATENT SPECIFICATION

(11) **EP 2 931 336 B1**
(45) Date of publication and mention of the grant of the patent: **14.06.2017**
(21) Application number: 13823932.2
(22) Date of filing: 09.12.2013
(51) Int. Cl.: A61M 5/20, A61M 5/31, A61M 5/32

(54) **DETECTION DEVICE AND INJECTION DEVICE COMPRISING IT**
ERFASSUNGSEINRICHTUNG UND INJEKTIONSVORRICHTUNG DIE DIESE ENTHAELT
DISPOSITIF DE DÉTECTION ET DISPOSITIF D'INJECTION LE COMPRENANT

(30) Priority: 13.12.2012 US 201261736790 P
(43) Date of publication of application: 21.10.2015
(73) Proprietor: Medicom Innovation Partner a/s, 7600 Struer (DK)
(72) Inventor: POULSEN, Sven Erik, 7800 Skive (DK); PEDERSEN, Christian Vig, 8220 Brabrand (DK)
(74) Representative: Rottenberg, Annabell Simone
(86) International application number: PCT/DK2013/000086
(87) International publication number: WO 2014/090252

(56) References cited:
- EP-A2- 0 520 443
- WO-A2-2008/108886
- WO-A2-2011/056888
- WO-A2-2012/037428
- US-A- 5 860 961

## Description

### Field of invention

The present invention generally relates to a detection device configured to assist (intra muscular) injection of a fluid (e.g., drug or another solution). The present invention more particularly relates to an auto-injection device comprising a detection device configured to assist (intra muscular) injection of a fluid (e.g., drug or other solution) to a body, where it is desired that drug is not injected into the blood vessels of the patient, since this can have consequences both in terms of overall patient safety but also e.g. drug efficacy.

### Prior art

During intra muscular injection of drugs it is often essential that the drug is delivered into the appropriate tissue. A large number of drugs may not be injected into the blood of the patient, e.g. an artery or a vein. Accordingly, it is important to aspirate in order to make sure that the needle is not inserted into an artery or into a vein. If a needle has been inserted into an artery or into a vein, blood will be sucked into the needle during the aspiration.

Hypodermic syringes are widely used to deliver fluids to the body. It is known to have hypodermic syringes applicable for manual operation, however, it is also known to have hypodermic syringes configured to be used in automated injection approaches.

During manual injection by using a hypodermic syringe blood being aspirated into the needle can be visually detected. However, during automated injection or infusion there is need for another secure and efficient procedure to ensure that the needle tip is not positioned e.g. in a vein thereby leading to potential harmful or inefficacious drug delivery. Thus, when it comes to automated injection of a fluid to a body there is a need for a detection device configured to assist the automated injection in a manner in which it is ensured that the drug is not injected into a blood vessel.

It is an object of the present invention to provide an auto-injection device that can be used to control delivering of a fluid (e.g., drug or other solution or suspension) to a patient in a manner in which it is secured, that drug is not injected into a blood vessel.

It is also an object of the present invention to provide an auto-injection device capable of providing an automatic "air shot" and delivering a fluid to a patient in a manner in which it is ensured, that the drug is not injected into a blood vessel.

Documents WO 2012/037428 A2 and WO 2011/056888 A2 disclose injection devices with detection means.

### Summary of the invention

The object of the present invention can be achieved by an auto-injection device as defined in claim 1. Preferred embodiments are defined in the dependent sub claims and explained in the following description and illustrated in the accompanying drawings.

The auto-injection device according to the invention comprises a detection device configured to assist injection of a media by using a hypodermic syringe comprising a needle attached to a needle hub. The detection device comprises means for identifying the characteristics of the media entering the needle hub or being present in the needle hub.

Hereby the detection device can assist the automated injection in a manner in which it is ensured, that the drug is not injected into a blood vessel. If blood is detected while entering the needle hub or being present in the needle hub, further injection can be prevented. Thus the patient safety can be increased.

It is preferred that the detection device comprises means for identifying if a needle hub is attached to the hypodermic syringe and if a needle cover is attached to the needle hub.

It may be an advantage that the detection device comprises means for calibrating the light source and/or the detection section and/or control unit and hereby compensate for individual needle hub's optic properties, where the detection device is configured to identify the characteristics of the media entering the needle hub or being present in the needle hub by using at least one detection algorithm, where said calibration is carried out by providing an offset adjustment of the at least one detection algorithm by comparing one or more of the needle hub's optic properties with one or more predefined values.

In this way the most accurate detections can be carried out. The detection device can be used even if the components are not capable of being calibrated at the moment they are built into the detection device. When a calibration is carried out t would be possible to apply less accurate and thus cheaper detection means.

The calibration may be carried out by using any suitable calibration principle applicable.

Hereby it is possible to potentially enabling a more robust detection of the needle hub in various conditions.

The information provided by the detection device can be used to assist a further the injection process (preferably by using an auto-injection according to the invention) and make sure that each step of the injection process is carried out in a satisfactory way.

It may be an advantage that the detection device comprises means for identifying the colour and/or size and/or shape of the needle hub.

Hereby the detection device can be capable of checking/verifying if the correct needle hub is applied. It is possible to configure the detection device in a manner so that it generates an alarm or provides another type of signal incase that a wrong needle is attached to the hypodermic syringe. In this way the detection device can enhance the patient safety. The detection device comprises:
- one or more light source configured to irradiate the needle hub with light;
- one or more light detection member adapted to detect light;
- a control unit configured to identify the characteristics of the media entering the needle hub on the basis of light detected by the detection section.

A detection device of such type can be provided by using standard components available. Such detection device will moreover be capable of providing valid detections.

It may be beneficial that the detection device comprises means for calibrating the light source and/or the detection section and/or control unit according to characteristics of a number of predefined individual disposable needles.

It may be an advantage that the detection device comprises a control unit control unit configured to identify a media consisting of air, water, blood, a predefined type of drug or mix of blood a predefined drug.

Hereby the patient safety can be increased by using the detection device. The detection device can be used to secure that a sufficient air shot process has been carried out and that no blood has entered into the needle hub during aspiration by way of example. It is also possible to secure that there is a match between the drug to be injected and the needle attached to the hypodermic syringe. If something is wrong, the detection device can generate a warning signal (e.g. a sound signal or a visual signal presented on a display).

It may be advantageous that the detection device comprises means for optical detection of the colour of at least a portion of the hypodermic syringe and/or that the detection device comprises means for determining characteristics of the hypodermic syringe on the basis of the detected the colour.
It may be beneficial that the detection device comprises means for identifying if the media is oxidized blood or deoxidized blood.

Hereby the detection device can be used to determine if the needle has been stuck into an artery or into a vein.

It is possible to use the absorption spectra from the media in order to verify if the media is oxidized blood or deoxidized blood. Thus, it may be an advantage that detection device comprises means for identifying if the media is oxidized blood or deoxidized blood on the basis of the absorption spectra from the media.

It may be advantageous that the detection device comprises means for detection of shift from a first condition to a second condition, where a first media or a mix of several media is present in the needle hub in the first condition, where a change of the content within the needle hub takes place in the second condition.

Hereby it is achieved that the detection device can detect the conditional shift when drug enters the needle hub during an "air shot" and that the detection device can detect the shift if blood enters the needle hub during an aspiration procedure.

It may be beneficial that the light source is a wideband light source and that the detection device comprises at least one detection member configured to detect light within a number of predefined wavelength ranges, preferably within at least two different predefined wavelength ranges.

It may be beneficial that the detection device is configured to identify the characteristics of the media entering the needle hub independently on the color of the needle hub.

Hereby the detection device can be used independently on the type of disposable needle that is applied. However, it may be beneficial that the detection device is configured to gain information about the needle on the basis of the visual characteristic of the needle (e.g. the colour of the needle hub).

It may be an advantage that the light source is a light-emitting diode (LED) and the light detection member is a wideband receiver.

Hereby it is possible to detect light in a large range of wavelengths and hereby gather as much information as possible.

It may be beneficial that the light source generates light having a predetermined modulation, such as a substantially square pulse sequence.

It may be advantageous that the detection device comprises means for gating the one or more detection section(s) in such a manner that the signal-to-noise ratio of the of the detected light is enhanced.

Hereby, the influence of noise introduced by light from the surroundings may be reduced.

When the light source is a laser diode the modulation can be achieved by directly modulating the drive current to the laser diode

It may be an advantage that the light detection member is an image sensor or a photo sensor.

Hereby it is possible to provide an image of the needle hub. Information about the needle hub may further be displayed for the user of the device e.g. on a display.

It may be beneficial that the image sensor is a charge coupled device (CCD) image sensor or a complementary metal oxide semiconductor (CMOS) image sensor.

It may be preferred that the light detection member is configured to determine the intensity of the light, preferably at one or more predefined frequencies. Hereby, it is possible to use specific frequencies (and thus specific wavelengths) to take a closer look at light at specific relevant frequencies/wavelengths.

It may be an advantage that the detection device comprises a number of electrodes configured to measure the conductivity of the media being present in the needle hub or entering the needle hub. These electrodes may detect fluid electrical conductivity properties that are not delectable by using optical sensor means.

It is preferred that the detection device comprises a display.

Hereby the information can be visualized and presented to the user of the device. The display may be any suitable type of display having any suitable shape and size. The display may be integrated into the housing of the detection device or may be connected to the detection device.

It is preferred that the detection device comprises a display that is configured to show when a needle hub is attached to the hypodermic syringe and/or if a needle cover is attached to the hypodermic syringe and/or when the needle hub is comprises air and/or drug and/or blood.

Hereby the user of the detection device can continuously be updated and informed about the status e.g. of the needle contents.

It is preferred that the detection device comprises a housing provided with an opening configured to receive the needle hub of a hypodermic syringe, where a light source is provided at a first side of the opening and where a light detection member is provided at a second side of the opening.

Such embodiment is easy to use and easy to provide with disposable needles. Moreover, there is access to perform the desired optical measurements necessary to carry out the desired detections.

It may be beneficial that the light source comprises a laser diode.

It may be an advantage that the detection device comprises means for detection of a drug having a reflection coefficient that is significantly larger than the reflection coefficient of water.

It may be advantageous that the detection device comprises means for detection of a drug (e.g. in aqueous suspension) having a reflection coefficient at normal incidence of at least 70-95% such as 90%.

It may be beneficial that the detection device comprises means for determining the presence of a media within the needle hub even though the detected reflected light comprises light reflected by several layers provided between the light source and the needle hub.

It may be an advantage that the image sensor comprises an optical filter member. The filter member may be any suitable filter member.

It may be an advantage that the light source is configured to emit light of various wavelengths (e.g. 450-490 nm, 530-570 nm, 550-590 nm, 610-650 nm and 960-1000 nm). Hereby it is possible to design an intelligent way of providing information about the media e.g. by taking advantage of knowledge about the absorption spectra of specific fluids.

In one embodiment according to the invention the light source comprises a laser diode. This may be an advantage if light with a specific wavelength is required. Moreover, it is easy to modulate a laser diode. Laser diodes are completely solid state, and therefore no fragile glass elements or critical setup procedures are required. Additionally, application of a laser diode makes it possible to achieve a large signal-to-noise ratio even under presence of day light.

It may be beneficial that the detection device comprises at least one lens. It may be preferred that the detection device comprises at least one converging lens arranged to focus light, e.g. light that is detected by a detection section.

It may be an advantage that the light source generates light having a predetermined wavelength and any predetermined type modulation.

It may be beneficial that the predetermined modulation comprising a substantially square pulse sequence (a square wave pulse sequence).

The light emitted from the light source can be unmodulated light of any desired wavelength. However, it may be advantageous that the emitted light is modulated to allow reliable detection of light transmitted by the light source and reflected from, or transmitted through a needle hub, irrespective of the light from the surrounding.

By way of example, light with a wavelength of between about 400 and 1100 nm and a modulation frequency of about 1-10 kHz may be used. It is possible to achieve isolation from surrounding light by filtering the modulation out of the signal at the detection section(s).

Advantageously, the detection device comprises a housing that is configured to receive hypodermic syringes of different sizes or geometries. Hereby the detection device can be used in more situations.

It is preferred that the detection device comprises means for showing an image of a needle hub.

Hereby detection device can provide the operator of the device with visual inspection of needle hub. Visualization of the needle hub in the display can be used to facilitate assisted manual control. It is possible to apply a white light source and a CCD camera by way of example. The detection device may comprise a colour type user interface display.

The objects of the present invention can in particular be achieved by using an auto-injection device comprising a detection device according to the invention.

Accurate detection in itself is desirable; however, it is of great value to use the information from the detection device in an auto-injection device and hereby take advantage of the information during the injection process.

It is preferred that the auto-injection device comprises a detection device in which the light source and/or the light detection member are configured to be maintained in a fixed position relative to the needle hub when the needle hub is being moved.

Hereby it is ensured that the needle hub can be monitored independently on its position. This is important if one desires to detect if blood enters the needle hub during aspiration by way of example.
It may be an advantage that the light source and/or the light detection member are slidably arranged to the housing. Hereby the light source and/or the light detection member can be maintained in a fixed position relative to the needle hub when the needle hub is being moved.

It may be beneficial that the auto-injection device comprises a light source and/or the light detection member axially slidably arranged to the housing relative to the needle hub with or without a needle.

It is preferred that the auto-injection device comprises means for automatically stopping when blood is detected in the needle hub and/or means for automatically stopping when drug is detected in the needle hub.

Hereby unintended injections can be avoided and the use of the auto-injection device is eased.

It is preferred that the auto-injection device is configured to use the information detected by the detection device during the injection processes. Hereby the patient safety can be increased. By way of example it is preferred that the auto-injection device automatically stops the injection process if a wrong (unsuitable) needle is detected.

It may be an advantage that the light source and/or the light detection member are attached in a fixed position relative to the auto-injection device.

It may be advantageous that the auto-injection device comprises at least one lens arranged to focus light transmitted by a light source or arranged to focus light that is received by a detection section.

It may be an advantage that the at least one lens is integrated in the housing or attached to the housing of the auto-injection device.

In a system according to the invention comprises an auto-injection device according to the invention, where the auto-injection device comprises a protective sleeve configured to receive the cylindrical casing.

### Description of the Drawings

The invention will become more fully understood from the detailed description given herein below. The accompanying drawings are given by way of illustration only, and thus, they are not limitative of the present invention. In the accompanying drawings:
- Fig. 1: shows schematic views of an auto-injection device according to the invention;
- Fig. 2: shows two close-up views of a detection device according to the invention;
- Fig. 3: shows different views of a hypodermic syringe with and without a needle;
- Fig. 4: shows different views of a detection device according to the invention;
- Fig. 5: shows schematic views of two detection devices according to the invention;
- Fig. 6: shows schematic views of two detection devices according to the invention;
- Fig. 7: shows schematic views of two auto-injection devices according to the invention;
- Fig. 8: shows an auto-injection device according to the invention and a graph showing absorption spectra of blood and water;
- Fig. 9 a): shows a schematically view of a detection device according to the invention having means for detection of reflected light;

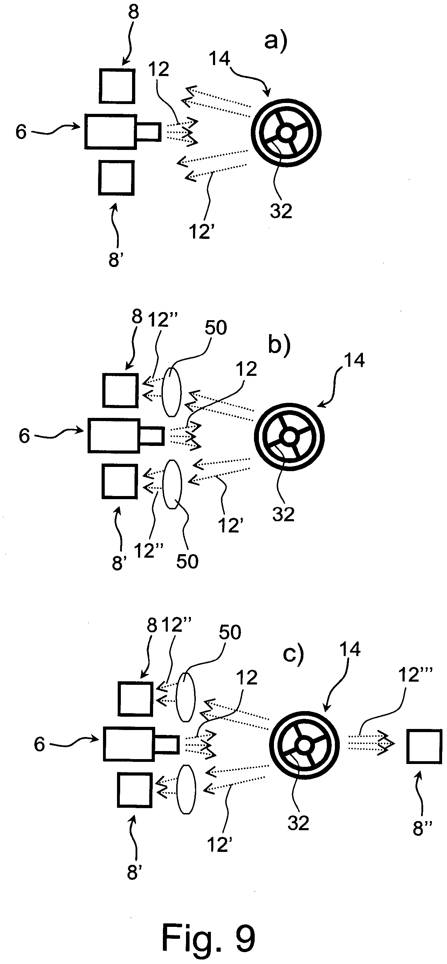

- Fig. 9 b): shows a schematically view of another detection device according to the invention having means for detection of reflected light;
- Fig. 9 c): shows a schematically view of another detection device according to the invention having means for detection of reflected light and means for detection of transmitted light;

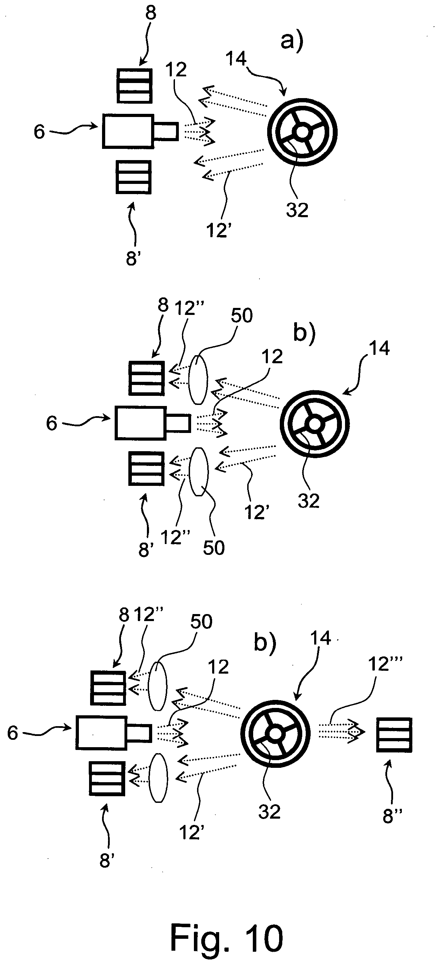

- Fig. 10 a): shows a schematically view of a detection device according to the invention having means for detection of reflected light;
- Fig. 10 b): shows a schematically view of another detection device according to the invention having means for detection of reflected light;
- Fig. 10 c): shows a schematically view of another detection device according to the invention having means for detection of reflected light and means for detection of transmitted light;

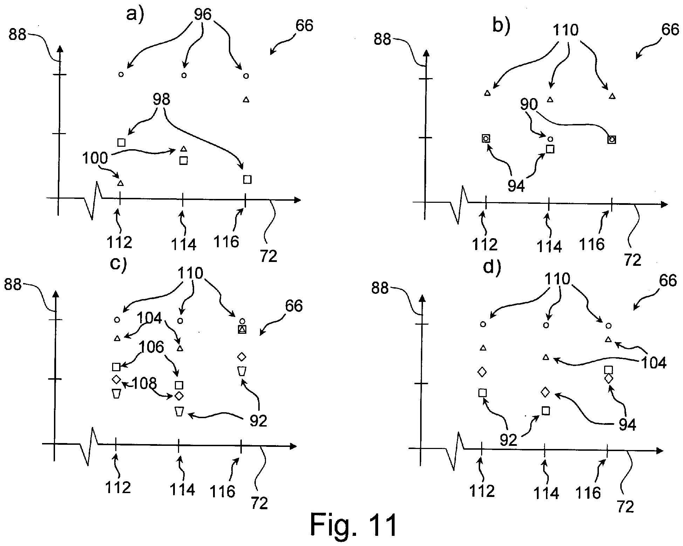

- Fig. 11 a): shows the relationship between the detected reflected light at three wavelengths for needle hub of different colours and a white label;
- Fig. 11 b): shows the relationship between the detected reflected light at three wavelengths for air, water and a drug, respectively;
- Fig. 11 c): shows the relationship between the detected reflected light at three wavelengths for a drug and four different mixtures of blood and drug, respectively;
- Fig. 11 d): shows the relationship between the detected reflected light at three wavelengths for a drug, water, blood and a mixtures of blood and drug, respectively;

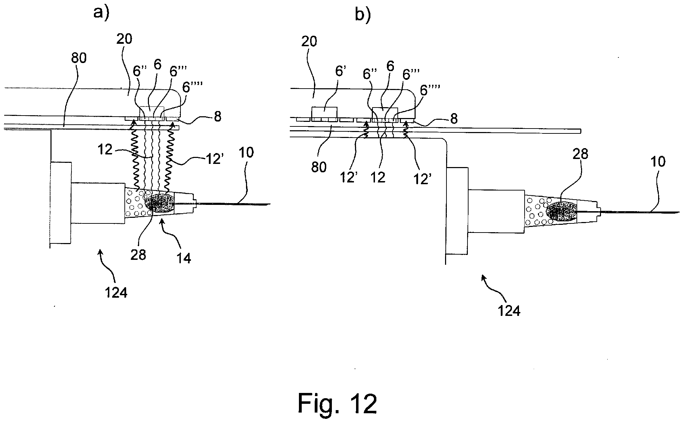

- Fig. 12 a): shows a schematically view of an auto-injection device according to the invention having means for detection of reflected light;
- Fig. 12 b): shows a schematically view of an auto-injection device according to the invention having means for detection of reflected light;

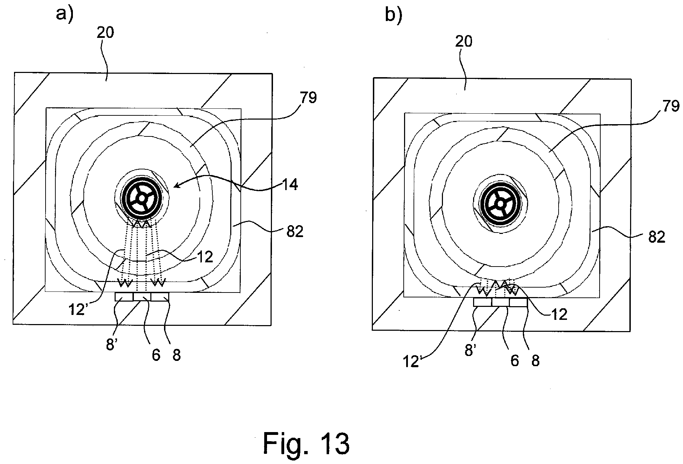

- Fig. 13 a): shows a schematically view of an auto-injection device according to the invention having means for detection of reflected light;
- Fig. 13 b): shows another schematically view of the auto-injection device shown in Fig. 13 a);

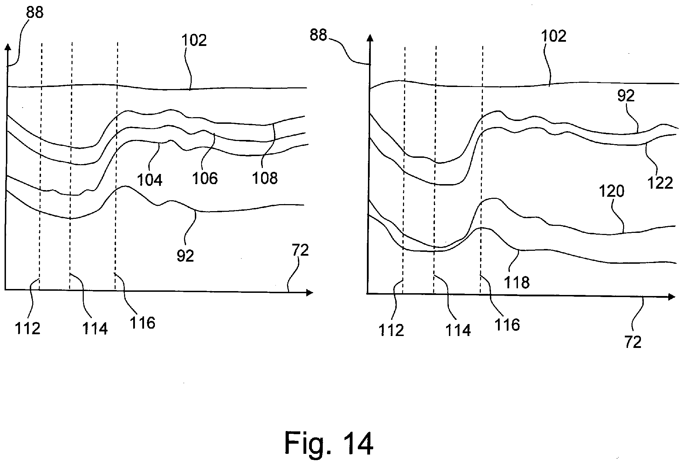

- Fig. 14 a): is a graph showing the relationship between the detected reflected light as function of the wavelengths for blood, a drug, and different mixtures of blood and drug, respectively;
- Fig. 14 b): is a graph showing the relationship between the detected reflected light as function of the wavelengths for blood, a drug, water, blood and different mixtures of blood and water, respectively;

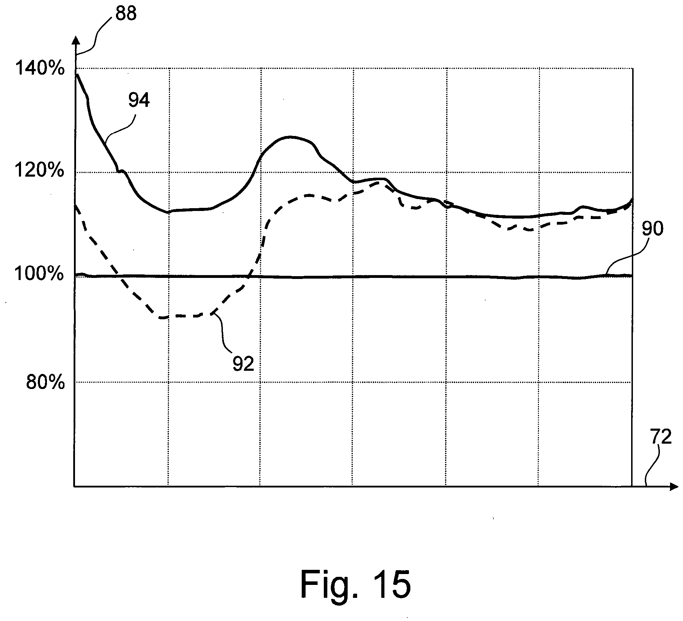

- Fig. 15: is a graph showing the relationship between the detected reflected light as function of the wavelength for blood, water and air, respectively and

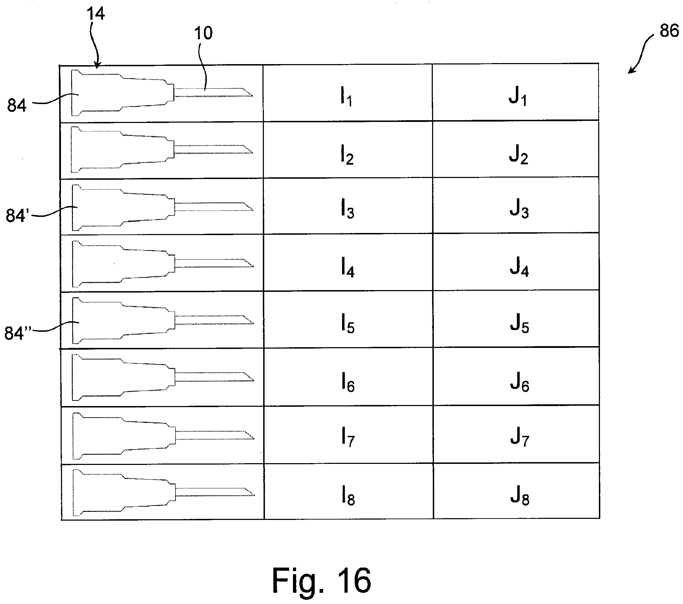

- Fig. 16: is a table showing the relationship between the colour code of a needle hub and the length and diameter of the needle.

### Detailed description of the invention

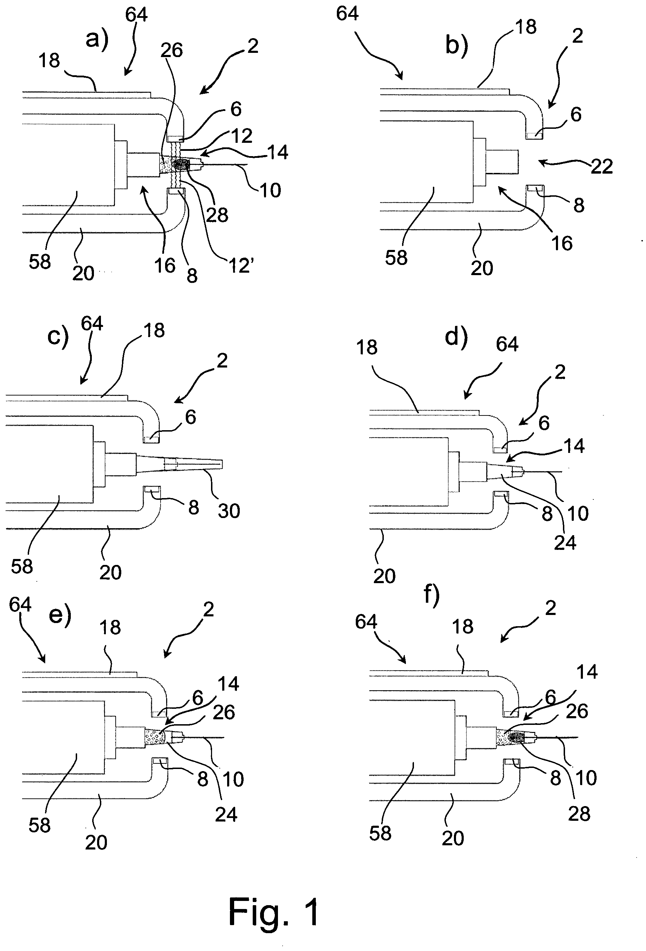

Referring now in detail to the drawings for the purpose of illustrating preferred embodiments of the present invention, an auto-injector 64 according to the present invention is illustrated in Fig. 1.

Fig. 1 a) is a schematic view of an auto-injector 64 according to the invention. The auto-injector 64 comprises an elongate housing 20 onto which, a display 18 is attached. A base member 58 is arranged in the housing 20 and a hypodermic syringe 16 is attached to the base member 58. A light source 6 and a detection section 8 are integrated into the housing 20. The light source 6 may be any suitable type of light source capable of transmitting light 12 of the desired intensity and spectrum.

The detection section 8 may be any suitable member capable of receiving light 12'. The light source 6 may be a light-emitting diode (LED) and the detection section 8 may be a 2-d optical sensor such as an image sensor 8 (e.g. a charge coupled device (CCD) image sensor or a complementary metal oxide semiconductor (CMOS) image sensor) or a photo sensor 8.

The hypodermic syringe 16 comprises a needle hub 14 with a needle 10. It can be seen that a drug 26 has been pumped into the needle hub 14. However, blood 28 has entered the needle hub 14 through the needle 10 (e.g. during aspiration). Light transmitted from the light 12 source 6 is directed towards the needle hub 14 of the hypodermic syringe 16. The fluid 26, 28 in the needle hub 14 attenuates the light 12 by absorption, reflection and scattering depending on the characteristics of the needle hub 14 and the fluid 26, 28 within it.

Accordingly, information about the transmitted light 12 and the detected light 12' (detected by the detection section 8) can be used to identify the fluid 26, 28 in the needle hub 14.

The base member 58 may comprise several mechanical means configured to carry out translation of the hypodermic syringe 16 (e.g. during injection and aspiration) and means for injecting a fluid 26 through needle 10 of the hypodermic syringe 16.

The display 18 may be configured to indicate various information, such as the status of the auto-injector 64 during an automated injection process. Preferably, the display 18 is configured to indicate: if a needle 10 with a needle hub 14 is attached to the hypodermic syringe 16; if the needle hub 14 is air filled; if the needle hub 14 is filled with drug 26 and if blood 28 is present in the needle hub 14.

Fig. 1 b) illustrates a schematic view of an auto-injector 64 according to the invention. The auto-injector 64 is almost similar to the one shown in Fig. 1a). However, the needle hub 14 with the needle 10 has been removed. It is preferred that the auto-injector 64 is capable of detecting that no needle hub 14 is attached to the hypodermic syringe 16. In a preferred embodiment according to the invention this information will be given by using the display 18. It is preferred that the information is automatically integrated with the control logics to support process control. Thus, in a preferred embodiment according to the invention the auto-injector 64 is configured to control the auto-injector 64 on the basis of data from the detection section 8.

Fig. 1 c) illustrates the auto-injector 64 shown in Fig. 1 a) and in Fig. 1 b). A needle cover 30 is attached to the needle hub 14 in order to protect against the needle 10. It is preferred that the auto-injector 64 is capable of detecting that a needle cover 30 is attached to the needle hub 14. It is preferred that this information can be indicated on the display 18 in order to assist the user of the auto-injector 64. However, it is preferred that the auto-injector 64 is configured to be automatically controlled by a control unit (not shown) using this information. By way of example it is preferred that the control unit is adapted to stop injection of a drug if blood 28 is detected in the needle hub 14 during aspiration. Similarly, it is preferred that the control unit is adapted to stop the injection process if an unknown (and unsuitable) needle hub 14 is detected. In this way the safety and usability of the auto-injector 64 can be increased.

It is possible that the air-filled needle hub 14 with the needle cover 30 can be used to carry out a calibration process before the needle hub 14 is filled with drug 26 or blood 28. Information about the transmitted light 12 and the detected light 12' in an arrangement in which the needle hub 14 is covered by a needle cover 30 may be valuable.

In Fig. 1 d) the needle cover 30 has been removed from the needle hub 14 and hence the needle hub 14 is air filled. This situation occurs before priming and it is preferred that the auto-injector 64 is capable of detecting that the needle hub 14 is filled with air 24. Preferably, this information can be indicated on the display 18. As previously mentioned it is preferred that the auto-injector 64 is configured to be automatically controlled on the basis of this information.

Fig. 1 e) illustrates a schematic view of the auto-injector 64 shown in Fig. 1d) during priming. It can be seen the auto-injector 64 that the drug 26 is pressed towards the needle 10, however, the tip of the needle hub 14 is still filled with air 24.

It is preferred that the detection device 2 is configured to determine when drug 26 enters the needle hub 14 and when the needle hub 14 is filled with drug 26. In this way it would be possible to minimize the amount of air which later in the injection process would be injected into the patient, thereby providing enhanced patient safety, and additionally avoid wasting of drug during the priming phase if manual priming would have led to unintended expelling of drug through the needle tip.

Fig. 1 f) illustrates the aspiration phase in which the hypodermic syringe 16 is reversed in order to verify if the needle 10 has penetrated a vein. In Fig. 1f) blood 28 is present in the needle hub 14. Accordingly, it may be concluded that the needle 10 is stuck into an artery or into a vein.

It is preferred that the auto-injector 64 is capable of detecting blood 28 as soon as the first quantity of blood 28 enters the needle hub 14. Preferably the display 18 is capable of showing or indicating this information. Again it is preferred that information about blood 28 in the needle hub 14 is used to control the auto-injector 64 automatically. This can be carried out through interaction with control logics. It is particularly preferred that the auto-injector 64 is capable of stopping injection of a drug in case that blood 28 is detected in the needle hub 14.

The auto-injector 64 shown in Fig. 1 is equipped with a detection device 2 capable of identifying if a media and/or a needle hub 14 with or without a needle cover 30 is present. This information can be used to guide the user of the auto-injector 64 and make the auto-injector 64 safer to use automatically - thereby limiting or entirely avoiding any manual user interaction.

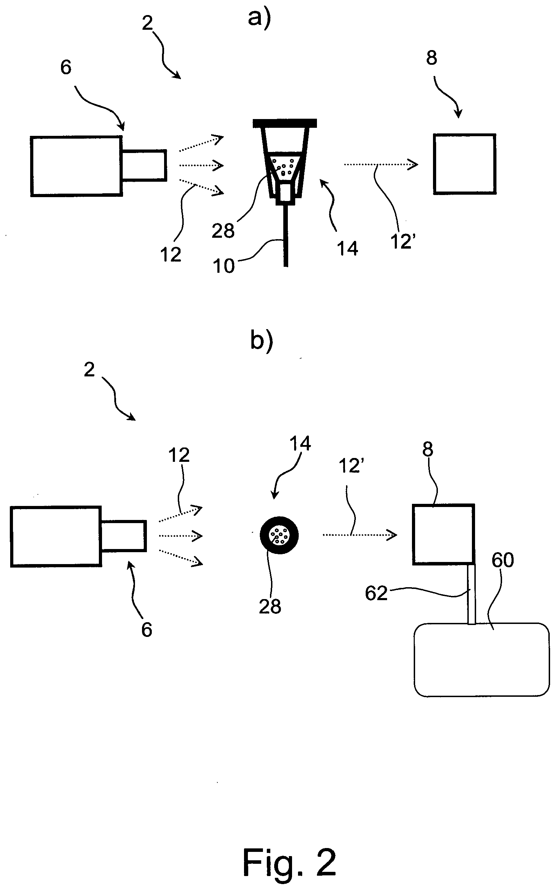

Fig. 2 a) illustrates a close-up side view of the basic elements of a detection device 2 according to the invention. The detection device 2 comprises a detection section 8 and a light source 6 in the form of a light emitting diode (LED) configured to emit light 12 of various wavelengths (e.g. 470 nm, 550 nm, 575 nm, 630 nm and 980 nm). The light 12 is directed towards a needle hub 14 comprising a needle 10.

The detection section 8 may be a 2-d optical sensor such as an image sensor 8 (such as a charge coupled device (CCD) image sensor or a complementary metal oxide semiconductor (CMOS) image sensor) or a photo sensor 8.

The needle hub 14 is filled with blood 28 and hence the light 12' received by the detection section 8 is influenced by the optical properties of the media in the needle hub 14 (in this case blood 28). Accordingly, when the expected/anticipated emitted light 12 is compared with the light received by the detection section 8, it is possible to determine what media that is present in the needle hub 14.

Fig. 2 b) illustrates another close-up view of the basic elements of a detection device 2 according to the invention. The detection device 2 comprises a detection section 8 that is electrically connected to a control unit 60 by means of a wire 62. The detection device 2 moreover comprises a light source 6 that may be a LED by way of example. It can be seen that the light 12 emitted from the light source 6 is directed towards the needle hub 14 and that the optical properties of the blood 28 in the needle hub 14 influences e.g. the intensity and spectral characteristics of the light 12' received by the detection section 8.

Information (data) from the detection section 8 is transmitted to the control unit 60 via the wired connection 62. However, of course a wireless connection is possible as an alternative. A connection may be provided between the transmitting light source 6 and the control unit 60. However, it may not be necessary to provide such connection due to the fact that, in most cases, the light source 6 emits light of predefined and known characteristics.

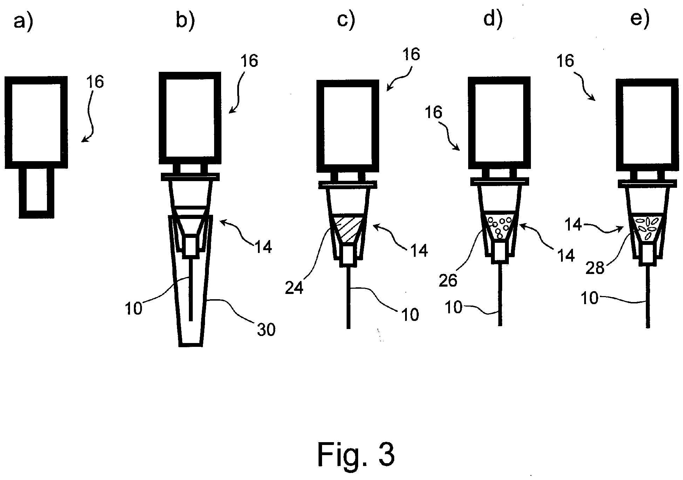

Fig. 3 illustrates five different side views of a hypodermic syringe 16. In Fig. 3 a) no needle hub 14 and thus no needle 10 is attached to the hypodermic syringe 16.

In Fig. 3 b) a needle hub with a needle 10 is attached to the hypodermic syringe 16. Moreover a needle cover 30 is attached to the needle hub 14 (for protection of the needle 10).

In Fig. 3 c) the needle cover 30 has been removed from the needle hub 14. The needle hub is filled with air 24 and no drug 26 has entered the needle hub 14.

In Fig. 3 d) the needle hub is filled with a drug 24. This situation corresponds to the state in which the priming process has been has been completed.

In Fig. 3 e) the needle hub 14 is filled with blood 28. This will occur then the needle 10 has been stuck unintended into a vein and aspiration has been performed whereby blood 28 from the vein has been withdrawn up into the needle hub 14.
The detection device 2 according to the invention is capable of identifying if a needle hub 14 is attached to the hypodermic syringe 16, identifying if a needle cover is present covering such needle and furthermore to identify what type of media (air, drug or blood) is present in the needle hub 14 by emitting light 12 towards the needle hub (if any) and analyse/detect the light 12' received by the detecting section 8 like illustrated in Fig. 1 and in Fig. 2. Preferably, the detection device 2 according to the invention is configured to distinguish between air, (different types of) drug and blood.

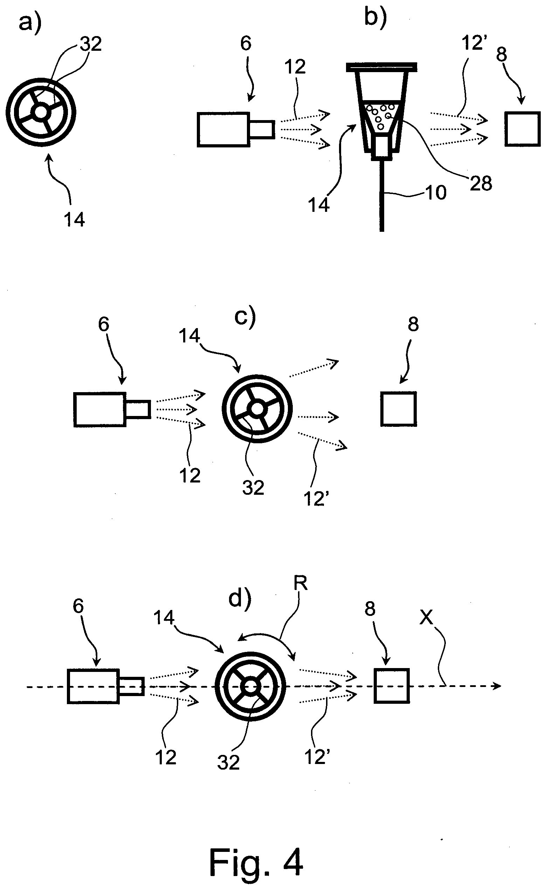

Fig. 4 a) illustrates a top view of a needle hub 14 of usable in an auto-injection device 64 according to the invention. The needle hub 14 comprises four symmetrically arranged radially extending vanes 32.

In Fig. 4 b), Fig. 4 c) and Fig. 4 d) it is indicated that the orientation of the vanes influences the intensity of the light detected by the receiving detection section 8. Therefore, it is preferred that the detection device 2 and the auto-injection device 64 according to the invention is configured to rotate the needle hub 14 until the e.g. highest intensity of light is detected by the detection section 8 or alternatively optimize the system for best possible detection signal-to-noise ratio.

In one embodiment according to the invention the needle hub 14 is automatically and progressively rotated while light 12 is emitted from the light source 6 until the maximum light intensity is received by the detection section 8. Such simple feedback mechanism is reliable and easy to implement. After such rotation optimization the angular position of the needle preferably remains unchanged throughout the entire remaining injection process in order to maintain stable detection bias conditions to allow enhanced sensitivity of various needle conditions.

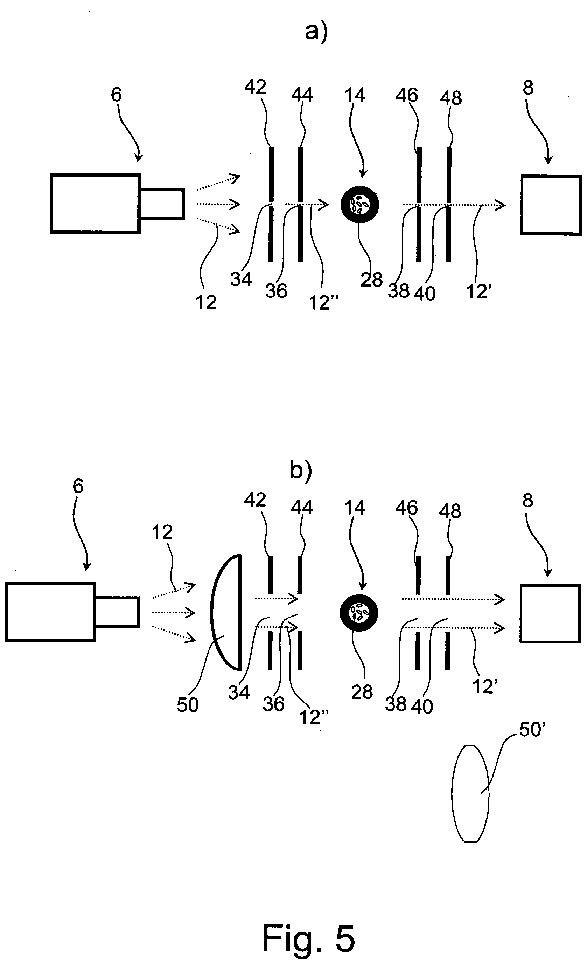

Fig. 5 a) illustrates one optical arrangements of a detection device according to the invention. The detection device 2 comprises a light source 6, a detection section 8, a first screen with an aperture 34, a second screen 44 with an aperture 36, a third screen 46 with an aperture 38 and a fourth screen 48 with an aperture 40. A needle hub 14 filled with blood 28 is arranged in the sampling zone between the light source 6 and the detection section 8.

The light source 6 is configured to emit light 12 towards the first screen through the aperture 34 and to further allow some of the light 12" to further pass through an aperture 36 in the second screen. The light 12' that both passes through the aperture 38 in the third screen and through the aperture 40 in the fourth screen 48 is detected by the detection section 8.

This optical measuring principle may be an advantage. The light 12 emitted from the light source 6 is focused at the needle hub 14. Hereby, the risk of "false" light (light not traveling through or reflected via the needle hub 14) entering the detection section 8 can be reduced.

Fig. 5 b) illustrates a schematically view of another optical arrangement of the elements of a detection device 2 according to the invention. The detection device 2 comprises a light source 6, a detection section 8, a first lens 50, optionally a second lens 50', a first screen 42 with an aperture 34, a second screen 44 with an aperture 36, a third screen 46 with an aperture 38 and a fourth screen 48 with an aperture 40. Like in Fig. 5 a) a needle hub 14 filled with blood 28 is arranged in the sampling zone between the light source 6 and the detection section 8.

The second lens 50' may be arranged between the fourth screen 48 and the detection section 8 in order to focus the light 12' before it is detected by the detection section 8.

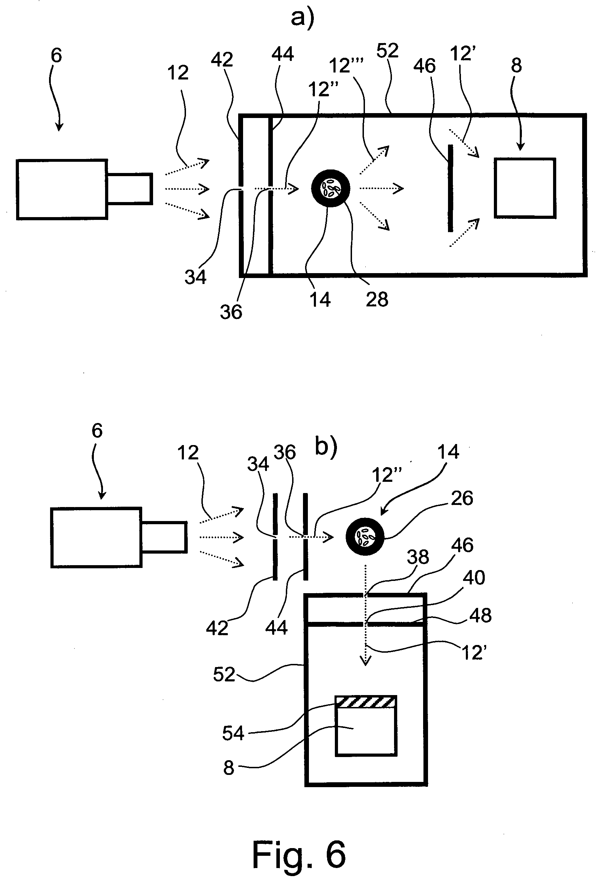

Fig. 6 a) illustrates a schematically view of an optical arrangement of the elements of a detection device 2 according to the invention, in which an alternative optical measuring principle is based on detection of indirect reflected and/or scattered light 12' from the needle hub 14.

The detection device 2 comprises a light source 6 and a detection section 8 arranged in a cover box 52. A first screen 42 with an aperture 34 and a second screen 44 with an aperture 36 are provided in the cover box 52. A third screen 46 is arranged between a needle hub 14 filled with blood 28 and the detection section 8.

Some of the light 12 emitted by the light source 6 passes through the apertures 34, 36 and further through the needle hub 14. The light 12" passing through the apertures 34, 36 is scattered by the blood filled needle hub 14. The light 12"' scattered by the blood filled needle hub 14 cannot directly be detected by the detection section 8, however, indirect light 12' originating from the light 12"' scattered by the blood filled needle hub 14 is detected by the detection section 8.

Fig. 6 b) illustrates a schematically view of another optical arrangement of the elements of a detection device 2 according to the invention.

The detection device 2 comprises a light source 6 and a detection section 8 arranged in a cover box 52. A first screen 42 with an aperture 34 and a second screen 44 with an aperture 36 are arranged between the light source 6 and a needle hub 14 filled with drug 26. The cover box 52 is arranged next to the needle hub 14. The cover box 52 comprises a third screen 46 with an aperture 38 and a fourth screen 48 with an aperture 40.

A 2-d optical sensor 8 is arranged in the cover box 52. The 2-d optical sensor 8 is provided with a filter 54.

Light 12 emitted from the light source 6 and some of this light 12" passes through the aperture 34 in the first screen 42 and through the aperture 36 in the second screen 44 and is directed towards the needle hub 14 filled with drug 26.

Some of the light 12 emitted by the light source 6 passes through the apertures 34, 36 and further towards the needle hub 14. The light 12" passing through the apertures 34, 36 is scattered by the drug filled needle hub 14. The light 12' scattered by the blood filled needle hub 14 can directly be detected by the detection section 8 when having entered the filter 54.

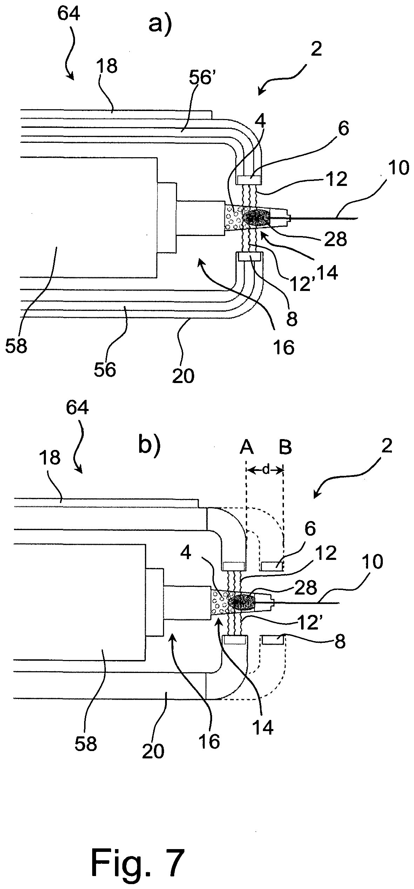

Fig. 7 a) illustrates one embodiment of an auto-injection device 64 according to the invention. The auto-injection device 64 corresponds essentially to the embodiment shown in Fig. 1 a), however, light guides 56, 56' are integrated in the wall of the housing 20. The first light guide 56' is connected to the light source 6, while the second light guide 56 is connected to the detection section 8.

The light guides 56, 56' are configured to guide light 12, 12'. The light 12 may be guided to the light source 6 e.g. from a LED arranged somewhere else within the housing 20. The light 12' detected by the detection section 8 may be guided to a control unit (not shown) for further analyses. The control unit is preferably configured to identify if a needle hub 14 is attached to the hypodermic syringe 16 and further to determine whether air 24, blood 28 or another media 4 is present in the needle hub 14.

Fig. 7 b) illustrates a preferred embodiment of an auto-injection device 64 according to the invention. The auto-injection device 64 corresponds essentially to the embodiment shown in Fig. 1 a). The light source 6 and the detection section 8 are slidably mounted to the housing 20 of the auto-injection device 64.

The distal portion of the housing wall is configured to be translated. In Fig. 7b) it is indicated that the light source 6 and the detection section 8 are arranged in a first position A, however it is also indicated (with a dotted line) that the light source 6 and the detection section 8 can be brought into another position B. The light source 6 and the detection section 8 are brought into the second position B by translating the distal portion of the housing 20.

Hereby the auto-injection device 64 is suitable for receiving needle hubs 14 of different lengths. Moreover, it is preferred that the auto-injection device 64 is configured to translate the light source 6 and the detection section 8 in order to keep them (the light source 6 and the detection section 8) in a fixed position relative to the needle hub 14. This means that the light source 6 and the detection section 8 are translated whenever the needle hub 14 is moved (e.g. during injection). Any suitable means may be used to perform this action.

It is indicated that the light source 6 and the detection section 8 are displaced a distance d from position A to position B. This translation may be provided by using an electric motor (not shown) during injection of a drug 26.

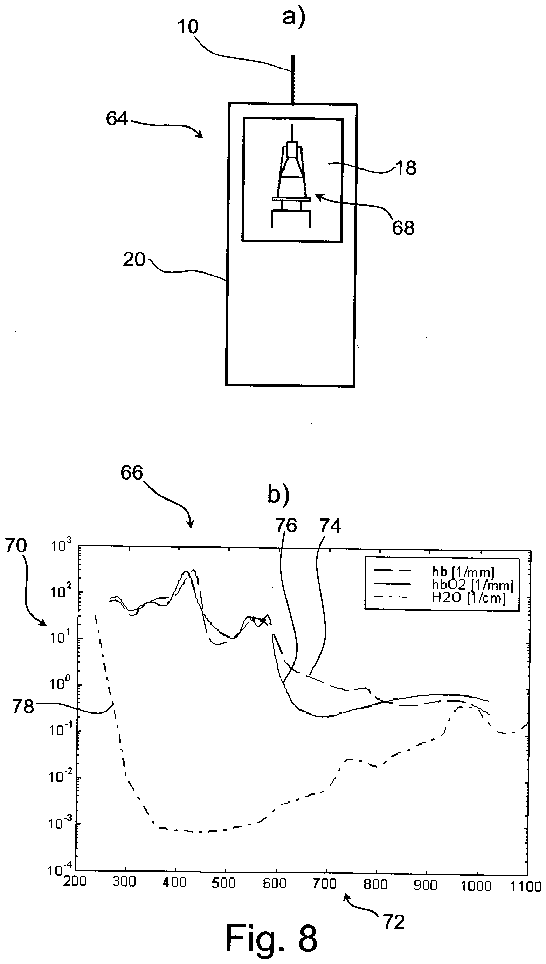

Fig. 8 a) illustrates a side view of an auto-injection device 64 according to the invention. The auto-injection device 64 has an elongate housing 20 with a display 18. A needle 10 is attached to the auto-injection device 64 and the display 18 shows an image 68 of the needle 10 attached to the auto-injection device 64.

The auto-injection device 64 is configured to supplement to the automated detection of needle hub 14 in various conditions (a condition with no needle hub 14 attached to the auto-injection device 64, a condition where a needle cover 30 is attached to the needle hub 14, a condition where the needle hub 14 is filled with either air 24, drug 26 or blood 28) is capable of capturing and showing an image 68 of the needle hub 14 on the display 18 in order to allow the operator of the auto-injection device 64 visual inspection of needle condition.

It is possible to use a white light source and a CCD camera and directly show an image 68 of the needle hub 14 on the device user interface display 18. It is preferred that the display 18 is a colour display 18.

Hereby visualization of the needle hub 14 in the display 18 can be used to facilitate assisted manual control.

Fig. 8 b) illustrates a graph 66 showing a typical blood absorption spectra 74, 76 compared to a water absorption spectrum. The graph 66 depicts the absorption 70 as function of the wavelength 72 (measured in nm). The absorption spectrum for oxidized blood 76, deoxidized blood 74 and water 78 can be compared by using the graph 66.

In the present invention this information can be used to detect if a media in a needle hub 14 is water or blood. By using knowledge about the absorption spectra of various fluids it is possible to provide the detection device according to the invention with an intelligent control unit capable of detect the presence of different fluids in the needle hub 14 and furthermore to distinguish between these fluids. It is by way of example possible to distinguish between oxidized blood and deoxidized blood or between drug and blood.

The media to be injected may be a drug in a solid-liquid biphasic liquid dosage form referred to as "suspension". The drug may also be a dissolved drug. The optical characteristics of the drug includes the drug's ability to absorb, reflect or transmit light of certain wavelengths.

The detection device according to the invention may detect light reflected by the drug if the drug is a reflective (e.g. a reflective suspension). The detection device according to the invention may detect light transmitted through the drug if the drug is a translucent medium (e.g. a reflective suspension).

Fig. 9 a) illustrates a schematically view of a detection device according to the invention. The detection device comprises a light source 6 that transmits light 12 towards a needle hub 14 arranged within the detection device. The needle hub 14 contains a media (such as air, drug, water or blood) and the needle hub 14 reflects light 12' that is received by a first detection section 8 and a second detection section 8' arranged adjacent to the light source 6.

Fig. 9 b) illustrates a schematically view of another detection device according to the invention. The detection device comprises a light source 6 that transmits light 12 towards a needle hub 14 that is arranged within the detection device. The needle hub 14 may contain air, drug, water or blood or a mixture of these. The needle hub 14 reflects light 12' that is received by a first and a second converging lens 50 arranged in front of a first detection section 8 and a second detection section 8', respectively.

The reflected light 12' is focused by the first and the second lens 50 and the focused light 12" is received by the first detection section 8 and the second detection section 8'. The light source 6 is arranged between the first detection section 8 and the second detection section 8'.

Fig. 9 c) illustrates a schematically view of another detection device according to the invention. The detection device comprises a light source 6 that transmits light 12 towards a needle hub 14 arranged within the detection device. The needle hub 14 contains a media such as air, drug, water or blood or a mixture of these. The needle hub 14 reflects light 12' that is focused by a first and a second converging lens 50 arranged in front of a first detection section 8 and a second detection section 8', respectively. The reflected light 12' is focused by the first and the second lens 50 and the focused light 12" is received by the first detection section 8 and the second detection section 8'.

Some of the transmitted light 12 is, however, transmitted through the needle hub 14. The light 12"' that is transmitted through the needle hub 14 is received by a detection section 8". A converging lens (not shown) may be arranged between the needle hub 14 and the detection section 8" for focusing the light 12".

Fig. 10 a) illustrates a schematically view of a detection device according to the invention almost similar to the one shown in Fig. 9 a). The only difference is that the a first detection section 8 and a second detection section 8' each comprises three separate detection members configured to detect light within three different predefined wavelength ranges.

The detection device comprises a light source 6 that transmits light 12 towards a needle hub 14 arranged within the detection device. The needle hub 14 contains a media (e.g. air, drug, water or blood). The needle hub 14 reflects light 12' that is received by a first detection section 8 and a second detection section 8' arranged adjacent to the light source 6.

Fig. 10 b) illustrates a schematically view of a detection device according to the invention almost similar to the one shown in Fig. 9 b). The first detection section 8 and the second detection section 8' each comprises three separate detection members configured to detect light within three different predefined wavelength ranges.

The detection device comprises a light source 6 that transmits light 12 towards a needle hub 14 that is arranged within the detection device. The needle hub 14 may contain a media such as air, drug, water or blood or a mixture of these. The needle hub 14 reflects light 12' received by a first and a second converging lens 50 arranged in front of a first detection section 8 and a second detection section 8', respectively.

The reflected light 12' is focused by the first and the second lens 50 and the focused light 12" is received by the first detection section 8 and the second detection section 8'. The light source 6 is arranged between the first detection section 8 and the second detection section 8'.

Fig. 10 c) illustrates a schematically view of another detection device according to the invention almost similar to the one shown in Fig. 9 c). Both the first detection section 8 and the second detection section 8' comprises three separate detection members configured to detect light within three different predefined wavelength ranges.

The detection device comprises a light source 6 that transmits light 12 towards a needle hub 14 arranged within the detection device. The needle hub 14 contains a media (e.g. air, drug, water or blood or a mixture of these). The needle hub 14 reflects light 12' that is focused by a first and a second converging lens 50 arranged in front of a first detection section 8 and a second detection section 8', respectively. The reflected light 12' is focused by the first and the second lens 50 and the focused light 12" is received by the first detection section 8 and the second detection section 8'.

Some of the transmitted light 12 is transmitted through the needle hub 14. The light 12"' that is transmitted through the needle hub 14 is received by a detection section 8". All though not shown a converging lens may be arranged between the needle hub 14 and the detection section 8" for focusing the light 12".

Fig. 11 illustrates four graphs 66 showing the relationship between the intensity of detected reflected from needle hubs of different colours and a white label at three wavelengths 112, 114, 116. The needle hubs have been illuminated by a light source as illustrated in Fig. 9 or in Fig. 10.

The graphs 66 depict the signal (intensity) of the reflected light as function of the wavelength 72 of the light. The first wavelengths 112, 114, 116 are 470 nm, 530 nm and 622 nm corresponding to the colours blue, green and orange, respectively.

In Fig. 11 a) the circles 96 represent the reflected light signal 88 from a white label. This signal is used as a reference. The squares 98 represent the reflected light from a blue needle hub, while the triangles 100 represent the reflected light from an orange needle hub.

It can be seen from Fig. 11 a) that the reflected light signal 88 from a blue needle hub is significantly different from the reflected light signal 88 from an orange needle hub. Accordingly, the reflected light signal 88 can be used to detect the colour of the needle hub.

When the detection device according to the invention is capable of verifying the colour of a needle hub, this information can be used to automatically providing information that relates to the needle hub colour such as dimensional information (length and diameter of the needle).

In Fig. 11 b) the circles 90 represent the reflected light signal 88 from an air-filled needle hub. This signal is used as a reference. The squares 94 represent the reflected light from a needle hub containing water, while the triangles 110 represent the reflected light from a needle hub containing a highly reflective drug.

It can be seen from Fig. 11 b) that the reflected light signal 110 from a drug-filled needle hub is significantly larger than the reflected light signals 90, 94 from a needle hub filled with air and water, respectively. Accordingly, the reflected light signal 88 can be used to detect the presence of drug in the needle hub during an automatic "air shot" procedure. As soon as drug is present a shift in the detected reflected light will be seen.

In Fig. 11 c) the circles 110 represent the reflected light signal 88 from a drug-filled needle hub (highly reflective drug). This signal is used as a reference. The triangles 104 represent the reflected light from a needle hub containing a mixture of blood (25%) and drug (75%), while the squares 106 represent the reflected light from a needle hub containing a mixture of blood (50%) and drug (50%). The diamonds 108 represent the reflected light from a needle hub containing a mixture of blood (25%) and drug (75%). The truncated cones represent the reflected light from a blood-filled needle hub.

Fig. 11 c) illustrates that the detection device according to the invention is capable of differentiating between blood-drug-mixtures of different drug content.

In Fig. 11 d) the circles 110 represent the reflected light signal 88 from a needle hub filled with a highly reflective drug. This signal is used as a reference. The triangles 104 represent the reflected light from a needle hub containing a mixture of blood, while the diamonds 94 represent the reflected light from a needle hub containing water. The squares 92 represent the reflected light from a needle hub containing blood.

Fig. 12 a) illustrates a schematically close-up view of an auto-injection device according to the invention. The auto-injection device comprises a housing 20 with a build-in light source 6 and two build-in detection sections 8 configured to detect light reflected light 12'.

A syringe 124 with a needle hub 14 having a needle 10 is arranged in the auto-injection device. Blood 28 has entered the needle hub 14 through the needle 10. The syringe 124 is arranged within a disposable casing 80 that encloses the syringe in order to avoid contamination of the auto-injection device.

The light source 6 transmits light 12 towards the needle hub 14. The light source 6 comprises three separate light source members 6", 6"', 6"" each transmitting light of different wavelengths (e.g. 470 nm, 530 nm and 622 nm).

Some of the transmitted light 12 is reflected by the needle hub and the media 28 contained by the needle hub 14. The reflected light 12' is detected by the build-in.

Fig. 12 b) illustrates another schematically close-up view of an auto-injection device according to the invention.

The auto-injection device comprises a housing 20 with a first light source 6 and a second light source 6'. The housing 20 also comprises detection sections 8 arranged and configured to detect light reflected light 12'.

A syringe 124 with a needle hub 14 having a needle 10 is partly arranged in the auto-injection device. The syringe 124 is arranged within a disposable casing 80 encloses the syringe in order to avoid contamination of the auto-injection device.

The first light source 6 transmits light 12 towards the syringe 124. The light source 6 comprises three separate members 6', 6", 6"' each transmitting light of different wavelengths such as 470 nm, 530 nm and 622 nm. Light 12' reflected by the syringe 124 is detected by the syringe 124.

Accordingly, the auto-injection device according to the invention is capable of detecting characteristics (e.g. the colour) of the syringe 124.

Fig. 13 a) illustrates a schematically cross-sectional view of an auto-injection device according to the invention. The auto-injection device comprises a housing 20 with a build-in light source 6 and two build-in detection sections 8, 8'.

It can be seen that some of the light 12 transmitted from the light source 6 is reflected by the needle hub 14. Reflected light 12' is detected by detection sections 8, 8' provided adjacent to the light source 6.

The auto-injection device according to the invention is configured to transmit light 12 through a sleeve 82, a casing 79 and the outer layer of the needle hub 14 and to detect light reflected by the media in the needle hub 14. The reflected light 12' represents light reflected by the layers of the sleeve 82, the casing 79 and the outer layer of the needle hub 14 as well as the reflection from the content of the needle hub 14.

Thus, the auto-injection device according to the invention is capable of determining the media within the needle hub 14 even though the detected reflected light 12' comprises components from the layers of the sleeve 82, the casing 79 as well as the outer layer of the needle hub 14.

Fig. 13 b) illustrates a schematically view of the auto-injection device shown in Fig. 13 a). The light source transmits light 12 towards the casing 79 and the casing 79 reflects the transmitted light 12.

The reflected light 12' is detected by a first detection section 8 and by a second detection section 8'.
This setup may be an advantage when the auto-injection device is applied to detect the colour of the casing 79. The colour of the casing 79 can be used to verify the dose of a drug.

Fig. 14 a) is a graph showing the relationship between the detected reflected light 88 as function of the wavelengths 72 for blood 92, a drug highly reflective drug used as reference 102, and different mixtures 104, 106, 108 of blood and drug, respectively;

The reflection curve of the reflective drug is used a reference 102. The curve 108 represents the detected reflected light when the detection device according to the invention has been applied to detect light reflected from a mixture of drug (75%) and blood (25%).

The curve 106 represents the detected reflected light when the detection device according to the invention has been applied to detect light reflected from a mixture of drug (50%) and blood (50%), while the curve 108 represents the detected reflected light when the detection device according to the invention has been applied to detect light reflected from a mixture of drug (25%) and blood (75%).

The curve 92 represents the detected reflected light when the detection device according to the invention has been used to detect light reflected from a blood-filled needle hub.

Three wavelengths 112, 114, 116 are indicated with dotted lines. These wavelengths 112, 114, 116 may be 470 nm, 530 nm and 622 nm corresponding to the colours blue, green and orange, respectively. The intersection points of the dotted lines and curves 92, 102, 104, 106, 108 are shown in Fig. 11 c).

Fig. 14 b) is a graph showing the relationship between the detected reflected light 88 as function of the wavelengths 72 for a drug highly reflective drug used as reference 102, blood 92, and different mixtures 118, 120, 122 of blood and water, respectively;

The reflection curve of the reflective drug is used a reference 102. The curve 118 represents the detected reflected light when the detection device according to the invention has been used to detect light reflected from a mixture of blood (25%) and water (75%), while the curve 92 represents the detected reflected light from blood.

The curve 120 represents the detected reflected light when the detection device according to the invention has been used to detect light reflected from a mixture of blood (50%) and water (50%), while the curve 122 represents the detected reflected light from a mixture of blood (75%) and water (25%).

Three wavelengths 112, 114, 116 are indicated with dotted lines. These wavelengths 112, 114, 116 corresponds to 470 nm, 530 nm and 622 nm corresponding to the colours blue, green and orange, respectively.

Fig. 15 is a graph showing the relationship between the detected reflected light 88 as function of the wavelength 72 for blood 92, water 94 and air 90, respectively in a setup in which a detection device according to the invention has been used to detect reflected light.

The reflection curve 90 represents the detected reflected light when the detection device according to the invention has been used to detect light reflected from an air-filled needle hub. The reflection of light may be caused a different number of layers (see Fig. 13) arranged between the light source and the content of the needle hub.

Fig. 15 illustrates that significantly different reflection signals 88 can be achieved (when comparing air, water and blood) by choosing specific wavelengths.

In one embodiment according to the invention these wavelengths are 470 nm, 530 nm and 622 nm.
Fig. 16 is a table 86 showing the relationship between the colour code 84, 84', 84" of a needle hub 14 and the corresponding length and diameter of the needle 10.

The table 86 comprises eight rows each having three columns. The first column comprises a colour code 84, 84', 84". The second column comprises information I₁, I₂, I₃, I₄, I₅, I₆, I₇, I₈, about the length of the needle 10, while the third column comprises information J₁, J₂, J₃, J₄, J₅, J₆, J₇, J₈, about the diameter of the needle 10.

The detection device according to the invention comprises storage means for storing information of the type indicated in Fig. 16. The detection device according to the invention is configured to determine the colour (code) 84, 84', 84" of a needle hub 14 and to verify the characteristics of the needle 10 on the basis of the colour (code) 84, 84', 84" of a needle hub 14.

This information may be applied to control the auto-injection device according to the invention in order to make sure that injecting is carried out at the proper depth.

### List of reference numerals

- 2: - Detection device
- 4: - Media
- 6, 6': - Light source
- 6", 6"', 6"": - Light source member
- 8, 8', 8": - Detection section
- 10: - Needle
- 12, 12', 12", 12"': - Light
- 14: - Needle hub
- 16: - Hypodermic syringe
- 18: - Display
- 20: - Housing
- 22: - Opening
- 24: - Air
- 26: - Drug
- 28: - Blood
- 30: - Needle cover
- 32: - Needle hub vane
- 34, 36, 38, 40: - Aperture
- 42, 44, 46, 48: - Screen
- 50, 50': - Lens
- 52: - Cover box
- 54: - Filter
- 56, 56': - Light guide
- 58: - Base member
- 60: - Control unit
- 62: - Wire
- 64: - Auto-injection device
- 66: - Graph
- 68: - Image
- 70: - Absorption
- 72: - Wavelength
- 74: - Absorption spectrum for blood
- 76: - Absorption spectrum for blood
- 78: - Absorption spectrum for water
- 79: - Casing
- 80: - Casing
- 82: - Sleeve
- 84, 84', 84": - Colour code
- 86: - Table
- 88: - Signal
- 90: - Air
- 92: - Blood
- 94: - Water
- 96: - With label
- 98: - Blue needle hub
- 100: - Orange needle hub
- 102: - Reference
- 104, 106, 108: - Mixture of drug and blood
- 110: - Drug
- 112, 114, 116: - Wavelength
- 118, 120, 122: - mixture of blood and water
- 124: - Syringe
- X: - Axis
- R: - Rotational direction
- A, B: - Position
- d: - Distance
- I₁, I₂, I₃, I₄, I₅, I₆, I₇, Is: - Information
- J₁, J₂, J₃, J₄, J₅, J₆, J₇, J₈: - Information

## Claims

1. An auto-injection device (64) comprising a detection device (2) configured to assist injection of a media (4, 26) by using a hypodermic syringe (16) comprising a needle (10) attached to a needle hub (14), wherein the detection device (2) comprises means (6, 8, 54, 56, 56', 60) for identifying the characteristics of the media (4, 26) entering the needle hub (14) or being present in the needle hub (14), **characterised in that** the detection device (2) comprises:
- one or more light sources (6) configured to irradiate the needle hub (14) with light;
- a light detection member (8) adapted to detect light (12);
- a control unit (60) configured to identify the characteristics of the media (4) entering the needle hub (14) on the basis of light detected by the detection section (8).

2. An auto-injection device (64) according to claim 1, **wherein** the detection device (2) comprises means (6, 8, 54, 56, 56', 60) for identifying if a needle hub (14) is attached to the hypodermic syringe (16) and/or if a needle cover (30) is attached to the needle hub (14).

3. An auto-injection device (64) according to claim 2, **wherein** the detection device (2) comprises means (6, 8, 54, 56, 56', 60) for identifying the colour and/or size and/or shape of the needle hub (14).

4. An auto-injection device (64) according to one of the preceding claims, **wherein** the detection device (2) comprises means (6, 8) for optical detection of the colour of at least a portion of the hypodermic syringe (16) and/or that the detection device (2) comprises means (60) for determining characteristics of the hypodermic syringe (16) on the basis of the detected the colour (84, 84', 84").

5. An auto-injection device (64) according to one of the preceding claims, **wherein** the detection device (2) comprises at least one detection section (8) arranged in such a manner that it can detect transmitted light (12') and/or reflected light (12").

6. An auto-injection device (64) according to one of the preceding claims, **wherein** the detection device (2) comprises a control unit (60) configured to identify a media (4, 24, 26, 28) consisting of air, water, blood, a predefined type of drug or mix of blood a predefined drug.

7. An auto-injection device (64) according to one of the preceding claims, **wherein** the detection device (2) comprises means for detection of shift from a first condition to a second condition, where a first media (110) or a mix of several medias (110, 92) is present in the needle hub (14) in the first condition, where a change of the content within the needle hub (14) takes place in the second condition.

8. An auto-injection device (64) according to one of the preceding claims, **wherein** the light source (6) is a wideband light source (6) and that the detection device (2) comprises at least one detection member (8) configured to detect light (12, 12', 12") within a number of predefined wavelength ranges, preferably within at least two different predefined ranges.

9. An auto-injection device (64) according to one of the preceding claims, **wherein** the light source (6) generates light having a predetermined modulation, such as a substantially square pulse sequence.

10. An auto-injection device (64) according to one of the preceding claims, **wherein** the detection device (2) comprises means for gating the one or more detection section(s) (8, 8', 8") in such a manner that the signal-to-noise ratio of the of the detected light (12', 12", 12"') is enhanced.

11. An auto-injection device (64) according to one of the preceding claims, **wherein** the detection device (2) comprises a display (18) and that the display (18) is configured to show when a needle hub (14) is attached to the hypodermic syringe (16) and/or if a needle cover (30) is attached to the hypodermic syringe (16) and/or when the needle hub (14) comprises air (24) and/or drug (26) and/or blood (28).

12. An auto-injection device (64) according to one of the preceding claims, **wherein** the detection device (2) comprises means (6, 8) for detection of a drug having a reflection coefficient that is significantly larger than the reflection coefficient of water.

13. An auto-injection device (64) according to one of the preceding claims, **wherein** the light source (6) and/or the light detection member (8) are configured to be maintained in a fixed position relative to the needle hub (14) when the needle hub (14) is being moved, wherein the light source (6) and/or the light detection member (8) are axially slidably arranged to the housing (20) so that the light source (6) and/or the light detection member (8) can be maintained in a fixed position relative to the needle hub (14) when the needle hub (14) is being moved.

14. An auto-injection device (64) according to one of the preceding claims, **wherein** the auto-injection device (64) is configured to use the information detected by the detection device (2) during the injection processes, wherein the detection device (2) preferably comprises means for automatically stopping when blood (92) is detected in the needle hub (14) and/or means for automatically stopping when drug (110) is detected in the needle hub (14).

15. An auto-injection device (64) according to one of the claims, **wherein** the auto-injection device (64) comprises at least one lens (50, 50') arranged to focus light (12, 12', 12") transmitted by a light source (6) or arranged to focus light (12, 12', 12") that is received by a detection section (8, 8') wherein the at least one lens (50, 50') is integrated in the housing (20) or attached to the housing (20) of the auto-injection device (64).

## Patentansprüche

1. Automatische Injektionsvorrichtung (64) umfassend eine Erfassungsvorrichtung (2), die dazu ausgestaltet ist, die Injektion eines Mediums (4, 26) unter Verwendung einer hypodermischen Spritze (16) mit einer an einer Nadelnabe (14) befestigten Nadel (10) zu unterstützen, wobei die Erfassungsvorrichtung (2) Mittel (6, 8, 54, 56, 56', 60) zur Identifikation der Eigenschaften des Mediums (4, 26), das in die Nadelnabe (14) eintritt oder in der Nadelnabe (14) vorliegt, umfasst, **dadurch gekennzeichnet, dass** die Erfassungsvorrichtung (2) umfasst:
- eine oder mehrere Lichtquellen (6), die dazu ausgestaltet sind, die Nadelnabe (14) mit Licht zu bestrahlen;
- ein Lichterfassungselement (8), das dazu geeignet ist, Licht (12) zu erfassen;
- eine Steuereinheit (60), die dazu ausgestaltet ist, die Eigenschaften des Mediums (4), das in die Nadelnabe (14) eintritt, auf Grundlage von Licht, das durch den Erfassungsabschnitt (8) erfasst wird, zu identifizieren.

2. Automatische Injektionsvorrichtung (64) nach Anspruch 1, wobei die Erfassungsvorrichtung (2) Mittel (6, 8, 54, 56, 56', 60) zur Feststellung umfasst, ob eine Nadelnabe (14) an der hypodermischen Spritze (16) befestigt ist und/oder ob eine Nadelkappe (30) an der Nadelnabe (14) befestigt ist.

3. Automatische Injektionsvorrichtung (64) nach Anspruch 2, wobei die Erfassungsvorrichtung (2) Mittel (6, 8, 54, 56, 56', 60) zur Identifizierung der Farbe und/oder Größe und/oder Form der Nadelnabe (14) umfasst.

4. Automatische Injektionsvorrichtung (64) nach einem der vorhergehenden Ansprüche, wobei die Erfassungsvorrichtung (2) Mittel (6, 8) zur optischen Erfassung der Farbe zumindest eines Abschnitts der hypodermischen Spritze (16) umfasst, und/oder die Erfassungsvorrichtung (2) Mittel (60) zur Bestimmung von Eigenschaften der hypodermischen Spritze (16) auf Grundlage der erfassten Farbe (84, 84', 84") umfasst.

5. Automatische Injektionsvorrichtung (64) nach einem der vorhergehenden Ansprüche, wobei die Erfassungsvorrichtung (2) zumindest einen Erfassungsabschnitt (8) umfasst, der auf solche Weise angeordnet ist, dass er ausgestrahltes Licht (12') und/oder reflektiertes Licht (12") erfassen kann.

6. Automatische Injektionsvorrichtung (64) nach einem der vorhergehenden Ansprüche, wobei die Erfassungsvorrichtung (2) eine Steuereinheit (60) umfasst, die dazu ausgestaltet ist, ein Medium (4, 24, 26, 28) zu identifizieren, das aus Luft, Wasser, Blut, einem vorbestimmten Typ von Arzneimittel oder einer Mischung aus Blut und einem vorbestimmten Arzneimittel besteht.

7. Automatische Injektionsvorrichtung (64) nach einem der vorhergehenden Ansprüche, wobei die Erfassungsvorrichtung (2) Mittel zur Erfassung eines Übergangs von einer ersten Bedingung auf eine zweite Bedingung umfasst, wobei in der ersten Bedingung ein erstes Medium (110) oder eine Mischung aus mehreren Medien (110, 92) in der Nadelnabe (14) vorliegt, wobei eine Veränderung des Inhalts in der Nadelnabe (14) in der zweiten Bedingung erfolgt.

8. Automatische Injektionsvorrichtung (64) nach einem der vorhergehenden Ansprüche, wobei die Lichtquelle (6) eine Breitband-Lichtquelle (6) ist und die Erfassungsvorrichtung (2) zumindest ein Erfassungselement (8) umfasst, das dazu ausgestaltet ist, Licht (12, 12', 12") in einer Anzahl von Wellenlängenbereichen zu erfassen, vorzugsweise in zumindest zwei unterschiedlichen vordefinierten Bereichen.

9. Automatische Injektionsvorrichtung (64) nach einem der vorhergehenden Ansprüche, wobei die Lichtquelle (6) Licht mit einer vorbestimmten Modulation erzeugt, etwa einer Sequenz von im Wesentlichen rechteckigen Impulsen.

10. Automatische Injektionsvorrichtung (64) nach einem der vorhergehenden Ansprüche, wobei die Erfassungsvorrichtung (2) Mittel zur Auswertung der einen oder mehreren Erfassungsabschnitte (8, 8', 8") auf solche Weise umfasst, dass das Signal-/Rausch-Verhältnis des erfassten Lichts (12', 12", 12"') verbessert wird.

11. Automatische Injektionsvorrichtung (64) nach einem der vorhergehenden Ansprüche, wobei die Erfassungsvorrichtung (2) eine Anzeigevorrichtung (18) umfasst und die Anzeigevorrichtung (18) dazu ausgestaltet ist, anzuzeigen, ob eine Nadelnabe (14) an der hypodermischen Spritze (16) befestigt ist und/oder ob eine Nadelkappe (30) an der hypodermischen Spritze (16) befestigt ist und/oder ob die Nadelnabe (14) Luft (24) und/oder Arzneimittel (26) und/oder Blut (28) enthält.

12. Automatische Injektionsvorrichtung (64) nach einem der vorhergehenden Ansprüche, wobei die Erfassungsvorrichtung (2) Mittel (6, 8) zur Erfassung eines Arzneimittels mit einem Reflexionskoeffizienten umfasst, der wesentlich höher ist als der Reflexionskoeffizient von Wasser.

13. Automatische Injektionsvorrichtung (64) nach einem der vorhergehenden Ansprüche, wobei die Lichtquelle (6) und/oder das Lichterfassungselement (8) dazu ausgestaltet sind, in einer fixen Stellung relativ zu der Nadelnabe (14) gehalten zu werden, wenn die Nadelnabe (14) bewegt wird, wobei die Lichtquelle (6) und/oder das Lichterfassungselement (8) axial verschiebbar an dem Gehäuse (20) angeordnet ist/sind, so dass die Lichtquelle (6) und/oder das Lichterfassungselement (8) in einer fixen Stellung relativ zu der Nadelnabe (14) gehalten werden kann, wenn die Nadelnabe (14) bewegt wird.

14. Automatische Injektionsvorrichtung (64) nach einem der vorhergehenden Ansprüche, wobei die automatische Injektionsvorrichtung (64) dazu ausgestaltet ist, die Informationen, die durch die Erfassungsvorrichtung (2) erfasst wurden, während der Injektionsvorgänge zu verwenden, wobei die Erfassungsvorrichtung (2) vorzugsweise Mittel zum automatischen Stoppen umfasst, wenn Blut (92) in der Nadelnabe (14) erfasst wird, und/oder Mittel zum automatischen Stoppen, wenn Arzneimittel (110) in der Nadelnabe (14) erfasst wird.

15. Automatische Injektionsvorrichtung (64) nach einem der vorhergehenden Ansprüche, wobei die automatische Injektionsvorrichtung (64) zumindest eine Linse (50, 50') umfasst, die angeordnet ist, um Licht (12, 12', 12"), das von einer Lichtquelle (6) ausgestrahlt wird, zu bündeln, oder um Licht (12, 12', 12"), das durch einen Erfassungsabschnitt (8, 8') empfangen wird, zu bündeln, wobei die zumindest eine Linse (50, 50') in das Gehäuse (20) integriert oder an dem Gehäuse (20) der automatischen Injektionsvorrichtung (64) befestigt ist.

## Revendications

1. Dispositif d'auto-injection (64) comprenant un dispositif de détection (2) configuré pour aider à l'injection d'un milieu (4, 26) en utilisant une seringue hypodermique (16) comprenant une aiguille (10) attachée à un raccord d'aiguille (14), dans lequel le dispositif de détection (2) comprend des moyens (6, 8, 54, 56, 56', 60) pour identifier les caractéristiques du milieu (4, 26) entrant dans le raccord d'aiguille (14) ou étant présent dans le raccord d'aiguille (14), **caractérisé en ce que** le dispositif de détection (2) comprend :
- une ou plusieurs source(s) de lumière (6) configurée(s) pour irradier le raccord d'aiguille (14) par une lumière ;
- un élément de détection de lumière (8) adapté pour détecter la lumière (12) ;
- une unité de commande (60) configurée pour identifier les caractéristiques du milieu (4) entrant dans le raccord d'aiguille (14) sur la base de la lumière détectée par la section de détection (8).

2. Dispositif d'auto-injection (64) selon la revendication 1, dans lequel le dispositif de détection (2) comprend des moyens (6, 8, 54, 56, 56', 60) pour identifier si un raccord d'aiguille (14) est attaché à la seringue hypodermique (16) et/ou si un protecteur d'aiguille (30) est attaché au raccord d'aiguille (14).

3. Dispositif d'auto-injection (64) selon la revendication 2, dans lequel le dispositif de détection (2) comprend des moyens (6, 8, 54, 56, 56', 60) pour identifier la couleur et/ou la taille et/ou la forme du raccord d'aiguille (14).

4. Dispositif d'auto-injection (64) selon l'une des revendications précédentes, dans lequel le dispositif de détection (2) comprend des moyens (6, 8) pour la détection optique de la couleur d'au moins une partie de la seringue hypodermique (16) et/ou dans lequel le dispositif de détection (2) comprend un moyen (60) pour déterminer les caractéristiques de la seringue hypodermique (16) sur la base de la couleur détectée (84, 84', 84").

5. Dispositif d'auto-injection (64) selon l'une des revendications précédentes, dans lequel le dispositif de détection (2) comprend au moins une section de détection (8) agencée de manière à pouvoir détecter la lumière transmise (12') et/ou la lumière réfléchie (12").

6. Dispositif d'auto-injection (64) selon l'une des revendications précédentes, dans lequel le dispositif de détection (2) comprend une unité de commande (60) configurée pour identifier un milieu (4, 24, 26, 28) consistant en l'air, l'eau, du sang, un type de médicament prédéfini ou un mélange de sang et d'un médicament prédéfini.

7. Dispositif d'auto-injection (64) selon l'une des revendications précédentes, dans lequel le dispositif de détection (2) comprend un moyen pour détecter le passage d'une première condition à une deuxième condition, dans lequel un premier milieu (110) ou un mélange de plusieurs milieux (110, 92) est présent dans le raccord d'aiguille (14) dans la première condition, où un changement de contenu dans le raccord d'aiguille (14) a lieu dans la deuxième condition.

8. Dispositif d'auto-injection (64) selon l'une des revendications précédentes, dans lequel la source de lumière (6) est une source de lumière à large bande (6) et dans lequel le dispositif de détection (2) comprend au moins un élément de détection (8) configuré pour détecter la lumière (12, 12', 12") dans un certain nombre de plages de longueur d'onde prédéfinies, de préférence dans au moins deux plages prédéfinies différentes.

9. Dispositif d'auto-injection (64) selon l'une des revendications précédentes, dans lequel la source de lumière (6) génère une lumière ayant une modulation prédéterminée, telle qu'une séquence d'impulsions sensiblement rectangulaires.

10. Dispositif d'auto-injection (64) selon l'une des revendications précédentes, dans lequel le dispositif de détection (2) comprend un moyen pour déclencher une ou plusieurs section(s) de détection (8, 8', 8") de sorte que le rapport signal sur bruit de la lumière détectée (12', 12", 12"') soit amélioré.

11. Dispositif d'auto-injection (64) selon l'une des revendications précédentes, dans lequel le dispositif de détection (2) comprend un dispositif d'affichage (18) et dans lequel le dispositif d'affichage (18) est configuré pour montrer lorsqu'un raccord d'aiguille (14) est attaché à la seringue hypodermique (16) et/ou si un protecteur d'aiguille (30) est attaché à la seringue hypodermique (16) et/ou lorsque le raccord d'aiguille (14) comprend de l'air (24) et/ou un médicament (26) et/ou du sang (28).

12. Dispositif d'auto-injection (64) selon l'une des revendications précédentes, dans lequel le dispositif de détection (2) comprend des moyens (6, 8) pour la détection d'un médicament ayant un coefficient de réflexion qui est significativement plus grand que le coefficient de réflexion de l'eau.

13. Dispositif d'auto-injection (64) selon l'une des revendications précédentes, dans lequel la source de lumière (6) et/ou l'élément de détection de lumière (8) est/sont configuré(s) pour être maintenu(e)(s) dans une position fixe par rapport au raccord d'aiguille (14) lorsque le raccord d'aiguille (14) est déplacé, dans lequel la source de lumière (6) et/ou l'élément de détection de lumière (8) est/sont agencé(s) axialement de manière coulissante sur le boîtier (20) de sorte que la source de lumière (6) et/ou l'élément de détection de lumière (8) peut/puissent être maintenu(e)(s) dans une position fixe par rapport au raccord d'aiguille (14) lorsque le raccord d'aiguille (14) est déplacé.

14. Dispositif d'auto-injection (64) selon l'une des revendications précédentes, dans lequel le dispositif d'auto-injection (64) est configuré pour utiliser les informations détectées par le dispositif de détection (2) pendant les processus d'injection, dans lequel le dispositif de détection (2) comprend de préférence un moyen pour effectuer un arrêt automatiquement lorsque du sang (92) est détecté dans le raccord d'aiguille (14) et/ou un moyen pour effectuer un arrêt automatiquement lorsqu'un médicament (110) est détecté dans le raccord d'aiguille (14).

15. Dispositif d'auto-injection (64) selon l'une des revendications, dans lequel le dispositif d'auto-injection (64) comprend au moins une lentille (50, 50') agencée pour focaliser la lumière (12, 12', 12") transmise par une source de lumière (6) ou agencée pour focaliser la lumière (12, 12', 12") qui est reçue par une section de détection (8, 8'), dans lequel l'au moins une lentille (50, 50') est intégrée dans le boîtier (20) ou attachée au boîtier (20) du dispositif d'auto-injection (64).
